(19) Europäisches Patentamt / European Patent Office / Office européen des brevets

(11) **EP 2 550 912 A1**

(12) **EUROPEAN PATENT APPLICATION**

(43) Date of publication:
**30.01.2013 Bulletin 2013/05**

(51) Int Cl.:
*A61B 3/103* (2006.01)   *A61B 3/13* (2006.01)
*G02B 21/00* (2006.01)   *A61F 2/16* (2006.01)

(21) Application number: **12005109.9**

(22) Date of filing: **10.07.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **28.07.2011 JP 2011165328**

(71) Applicant: **Kabushiki Kaisha TOPCON
Tokyo 174-8580 (JP)**

(72) Inventors:
• **Kitajima, Nobuaki
Saitama 349-0212 (JP)**
• **Sato, Toshiaki
Tokyo (JP)**

(74) Representative: **Gagel, Roland
Patentanwalt Dr. Roland Gagel
Landsberger Strasse 480a
81241 München (DE)**

(54) **A microscope for ophthalmologic surgery**

(57)    A microscope for ophthalmologic surgery is provided which can measure astigmatic axis angles with high accuracy regardless of the drawing positions of the patient's eye in an image frame. Astigmatism distribution information 71 showing the distribution of the astigmatism parameters resulting from the power distribution of an imaging optical system at least is stored in the storage 70 of the microscope for ophthalmologic surgery 1. An astigmatism information calculating part 92 calculates astigmatism information based on images obtained by photographing the patient's eye E with an imaging element 56a while light from the group of LEDs 131-i is being projected. An astigmatism information correcting part 93 corrects this astigmatism information based on the astigmatism distribution information 71.

FIG. 6

EP 2 550 912 A1

## Description

[Field of the Invention]

The present invention relates to a microscope for ophthalmologic surgery.

[Background of the Invention]

A microscope for ophthalmologic surgery, which can measure the astigmatic axis angles of a patient's eye and project the measurement results onto the patient's eye, is known (Refer to Japanese published unexamined application 2011-110172. The contents of Japanese published unexamined application 2011-110172 will be invoked in the specifications). The microscope for ophthalmologic surgery projects multiple bright points arranged in a circle onto the patient's eye, calculates the astigmatic axis angle based on the arrangement of these projection images, and is used for transplant surgeries of toric IOL (Intraocular Lens) for correcting astigmatism.

To effectively correct astigmatism, not only the astigmatic degree but also the astigmatic axis angle is important. Consequently, it is necessary to adjust the orientation of the lens with high accuracy when transplanting the toric IOL. The microscope for ophthalmologic surgery in Japanese published unexamined application 2011-110172 satisfies such needs. Furthermore, it is also advantageous in that the traditional method that directly marks the astigmatic axis angle on the patient's eye does not have to be followed.

[Prior Art Documents]

[Patent Documents]

[Patent Document 1] Japanese published unexamined application 2011-110172

[Summary of the Invention]

[Problem to be Solved by the Invention]

As an aside, operators properly align the microscope with the patient's eye by hand during ophthalmologic surgery. This is different from ophthalmologic apparatuses (such as an auto refractometer) that conduct alignment automatically. Due to such circumstances, the patient's eye may be drawn to a position outside the center of image frames, potentially causing distortion on images in the microscope for ophthalmologic surgery. In this case, errors will mediate the measurement results of the astigmatic axis angles.

Furthermore, the instrument forming the projection images in Japanese published unexamined application 2011-110172 can be removed from the microscope itself and used with various microscopes. As a result, the measurement of astigmatic axes using the instrument is affected by the microscope optical system as well as the conditions of the camera. For example, the microscope optical system has a specific aberration and aspect ratios when capturing images with a camera vary. Differences in such conditions affect the measurement results of the astigmatic axis angle. Particularly, the impact on the measurement results differs in response to the drawing position of the patient's eye in the image frame.

The present invention was developed to solve said problems, with the intention of providing a microscope for ophthalmologic surgery that can measure astigmatic axis angles with high accuracy regardless of the drawing position of the patient's eye in an image frame.

[Means for Solving the Problem]

A microscope for ophthalmologic surgery according to claim 1 comprises: an optical system that includes an illumination optical system that illuminates the patient's eye and an imaging optical system that has an imaging element that photographs said illuminated patient's eye; a main body part that stores at least a part of said optical system; an attachment that is attached to said main body part and has multiple bright points arranged in a ring; a calculating part that calculates astigmatism information based on images obtained by photographing said patient's eye with said imaging element while said multiple bright points are being projected; a storage in which astigmatism distribution information that shows the distribution of astigmatism parameters resulting from the power distribution of said imaging optical system at least in advance; and a correcting part that corrects the astigmatism information calculated by said calculating part based on said astigmatism distribution information.
An invention according to claim 2 is the microscope for ophthalmologic surgery according to Claim 1, wherein said astigmatism information calculated by said calculating part includes the astigmatic axis angle, and further comprises a controller that specifies a bright point at a position corresponding to said corrected astigmatic axis angle from among said multiple bright points and makes the aspect of said specified bright point different from the aspect of other bright points.

An invention according to claim 3 is the microscope for ophthalmologic surgery according to Claim 1 or Claim 2, wherein said astigmatism parameters at each position in a frame of a photographed image by the imaging element is recorded in said astigmatism distribution information.

An invention according to claim 4 is the microscope for ophthalmologic surgery according to any one of Claims 1 through Claim 3, wherein information obtained by applying a least-squares method to the measurement results of said astigmatism parameters at multiple positions in a frame of a photographed image by said imaging element is recorded in said astigmatism distribution information.

An invention according to claim 5 is the microscope for ophthalmologic surgery according to any one of Claims 1 through Claim 4, further comprising a generating part that obtains, based on a photographed image by said imaging element, said astigmatism parameters at each of multiple positions in the relevant frame, and generates said astigmatism distribution information by applying the least-squares method to the obtained multiple astigmatism parameters.

An invention according to claim 6 is the microscope for ophthalmologic surgery according to Claim 5, wherein said generating part obtains multiple pairs of an astigmatic degree and an astigmatic axis angle as said multiple astigmatism parameters based on multiple said photographed images on which an image of an astigmatism-free schematic eye is drawn at each of said multiple positions while said multiple bright points are being projected.

An invention according to claim 7 is the microscope for ophthalmologic surgery according to Claim 6, wherein said generating part: obtains, with regard to each of said pair of the astigmatic degree and the astigmatic axis angle, two-dimensional orthogonal coordinates $(X_i, Y_i)$ corresponding to polar coordinates $(C_i, \theta_i)$ that treat said astigmatic degree $C_i$ as a radius vector and the doubled value $2\theta_i$ of said astigmatic axis angle $\theta_i$ as a polar angle; calculates a first approximation surface of nth order for partial or the entire said frame by applying the least-squares method to the multiple coordinates $X_i$ in the obtained multiple two-dimensional orthogonal coordinates $(X_i Y_i)$; and calculates the second approximation surface of nth order for partial or the entire said frame by applying the least-squares method to the multiple coordinates $Y_i$ in the multiple two-dimensional orthogonal coordinates $(X_i, Y_i)$, wherein said calculated first approximation surface of nth order and said second approximation surface of nth order are treated as said astigmatism distribution information.

An invention according to claim 8 is the microscope for ophthalmologic surgery according to Claim 7, wherein said calculating part calculates the astigmatic degree and the astigmatic axis angle as said astigmatism information, and said correcting part: obtains the two-dimensional orthogonal coordinates $(X_m, Y_m)$ corresponding to polar coordinates $(C, \theta)$ that treat the astigmatic degree C calculated by said calculating part as the radius vector and the doubled value $2\theta$ of the astigmatic axis angle $\theta$ as a polar angle; subtracts coordinates $X_c$ of said first approximation surface of nth order and coordinates $Y_c$ of said second approximation surface of nth order at the center position of an elliptic array of the images of said multiple bright points which are drawn in an image of said patient's eye obtained by said imaging element, from said coordinates $X_m$ and said coordinates $Y_m$, respectively; and obtains the astigmatic degree and the astigmatic axis angle by transforming the coordinates $X_m - X_c$ and the coordinates $Y_m - Y_c$ obtained from the subtraction into polar coordinates.

[Effect of the Invention]

The microscope for ophthalmologic surgery related to the present invention can correct measured values of the astigmatism information of the eyes of patients using information on astigmatism distribution, which shows the distribution of astigmatism parameters caused by the power distribution of an imaging optical system at least. Consequently, the astigmatic axis angles can be measured with high accuracy regardless of the drawing position of the patient's eye in an image frame.

[Brief Description of the Drawings]

Fig. 1 is a schematic drawing showing an example of the exterior configuration of the microscope for ophthalmologic surgery pertaining to the embodiment.
Fig. 2 is a schematic drawing showing an example of the configuration of a projection image forming part in the microscope for ophthalmologic surgery pertaining to the embodiment.
Fig. 3 is a schematic drawing showing an example of the configuration of a projection image forming part in the microscope for ophthalmologic surgery pertaining to the embodiment.
Fig. 4 is a schematic drawing showing an example of the configuration of an optical system in the microscope for ophthalmologic surgery pertaining to the embodiment.
Fig. 5 is a schematic drawing showing an example of the configuration of an optical system in the microscope for ophthalmologic surgery pertaining to the embodiment.
Fig. 6 is a schematic block diagram showing an example of the configuration of a control system in the microscope for ophthalmologic surgery pertaining to the embodiment.
Fig. 7 is a schematic drawing showing an example of a display screen in the microscope for ophthalmologic surgery pertaining to the embodiment.

[Mode for Carrying Out the Invention]

An example of an embodiment of the microscope for ophthalmologic surgery, as related to the present invention, will be explained in detail with reference to the diagrams.

[Configuration]

[Exterior configuration]

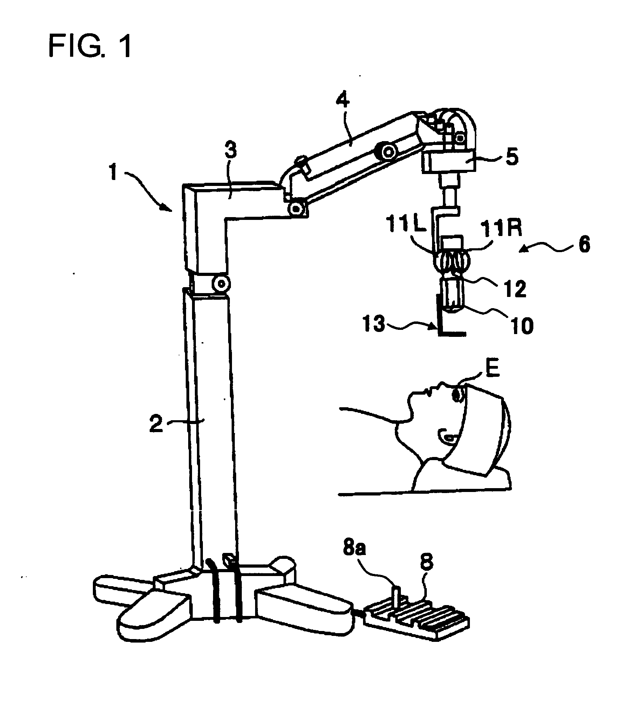

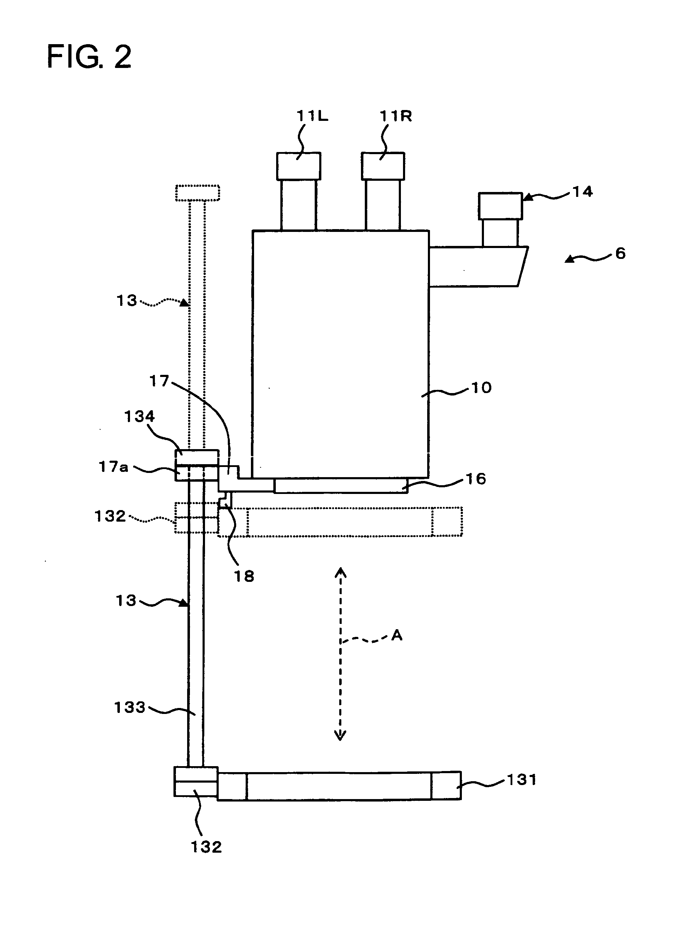

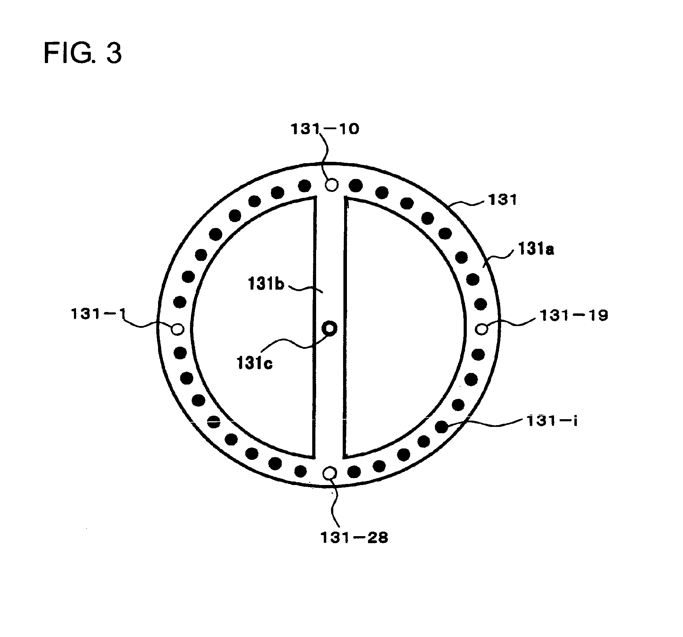

The exterior configuration of a microscope for ophthalmologic surgery 1, as related to the embodiment, will be explained with reference to Fig. 1. The microscope for ophthalmologic surgery 1 is composed of a pole 2, a first arm 3, a second arm 4, a driving device 5, a microscope 6, and a foot switch 8.
The driving device 5 is composed of an actuator such as a motor. The driving device 5 moves the microscope 6 up and down as well as horizontally, depending on the operation using an operating lever 8a of the foot switch 8. This makes it possible to move the microscope 6 3-dimensionally.
Various optical systems, drive systems, etc. are stored in a lens tube part 10 of the microscope 6. An inverter part 12 is provided in the top part of the lens tube part 10. When an observation image of a patient's eye is obtained as a reversed image, the inverter part 12 converts the observation image to an erect image. A left-and-right pair of eyepiece parts 11L and 11R is provided at the top part of the inverter part 12. An observer (such as an operator) can view the patient's eye with the both eyes by looking into the eyepiece parts 11 L and 11R. The microscope 6 is an example of the "main body."
The microscope for ophthalmologic surgery 1 has a projection image forming part 13. The projection image forming part 13 is removable from the microscope 6 and forms a specified projection image on the patient's eye E by projecting luminous flux onto the patient's eye E. The projection image forming part 13 is an example of an "attachment."
A configuration example of the projection image forming part 13 is shown in Fig. 2 and Fig. 3. The symbol 14 in Fig. 2 represents a photographing part. A TV camera 56, etc. that is discussed later is stored in the photographing part 14. Furthermore, Fig. 3 represents the configuration when a head part 131 of the projection image forming part 13 is viewed from the bottom (that is, from the side of the patient's eye E, in other words, from the side opposite to the lens tube part 10).
As indicated in Fig. 2 and Fig. 3, the head part 131 is a plate-like member. As indicated in Fig. 3, a toric periphery 131 a and a fixation-light-source holder 131b, which is bridged in the direction of the diameter of the periphery 131 a, are provided in the head part 131.
Multiple LEDs 131-i (i=1 to N) are provided on the bottom surface of the periphery 131a. A group of LED 131-i are placed in a ring. In this embodiment, 36 LEDs 131-i are provided at even intervals (N=36). That is, the group of LEDs 131-i are placed at intervals of 10 degrees with regard to the center position of the group of LEDs 131-i. In other words, when considering line segments that connect each LED 131-i and the center position, two line segments that relate to LED 131-i and the adjacent 131-(i+1) are crossed at the center position at an angle of 10°.
Each LED 131-i emits visible light. A group of LEDs 131-i can be composed such that all LEDs emit the same color and can also be composed to emit different colors. The latter case can be composed such that LEDs in the horizontal direction and the vertical direction in the group of LEDs 131-i output different colors from the LEDs in other directions. That is, it can be composed such that LEDs placed at astigmatic axis angles (astigmatic axis directions) 0 degrees, 90 degrees, 180 degrees, and 270 degrees (LED 131-1, 131-10, 131-19, 131-28 accordingly) output luminous flux of different colors from other LEDs 131-i (i≠1, 10, 19, 28). For example, red LEDs can be used for LEDs 131-1, 131-10, 131-19, 131-28 along with green LEDs for other LEDs 131-i (i≠1, 10, 19, 28). As a result, the horizontal direction and the vertical direction of the astigmatic axes can be easily identified.
Furthermore, only a part of LED 131-1, 131-10, 131-19, and 131-28 can be set up to output luminous flux of different colors from other LEDs. For example, only LED 131i corresponding to a 0 degree angle can be composed to output different colors than other LEDs (i≠1).
In addition, it is not necessary that all of the LEDs 131-1, 131-10, 131-19, and 131-28 are composed to output luminous flux of the same color (red in the example above). For example, red LEDs can be used for LEDs 131-1 and 131-19 while white LEDs can be used for LEDs 131-10 and 131-28 and green LEDs can be used for other LEDs 131-i ($i \neq 1, 10, 19, 28$). As a result, the horizontal direction and the vertical direction of the astigmatic axes can be easily identified.

Moreover, instead of making the horizontal direction and vertical direction identifiable by an output color of a light source such as above, other configurations can have a similar effect. For example, changing the brightness of the output light can make the directions identifiable.

A fixation LED 131 c for fixating the patient's eye E is provided on the bottom surface of the fixation-light-source holder 131b. The fixation LED 131 c emits visible light. The fixation LED 131c is, for example, placed at the above center position of the group of LEDs 131-i, which are placed in a ring.

The number of the fixation LED 131c is not limited to 1 but any number can be provided. Furthermore, the fixation LED 131 c can be composed so as to be movable.

Moreover, the light source used for the head part does not have to be an LED and any device that can output light may be used. Furthermore, the multiple light sources placed in a ring do not have to be placed at even intervals. Further, because Fig. 3 is a diagram of the bottom surface, the placement order of the group of LEDs 131-i is set up in the opposite direction (reverse direction) from the direction set up for a general astigmatic axis. As a result, corneal reflection lights of luminous fluxes that re output from the group of LEDs 131-i (Purkinje images) are observed or photographed as the direction set up for a general astigmatic axis.

In addition, though 36 light sources are placed in a ring in this embodiment, the quantity is also arbitrary. It should be noted that when measuring the astigmatic axis angle of the patient's eye E based on the Purkinje image of luminous fluxes that are output by the group of LEDs 131-i, it is desirable that the quantity of light sources, which can ensure precision and accuracy of the measurements, are provided. When adopting a configuration that does not measure the stigmatic axis angles, there is no limit to the quantity of light sources in this sense.

Furthermore, depending on the precision required when the operator visually recognizes the orientation of the astigmatic axis angle and the principal meridian of toric IOL, the quantity of light sources can be properly set. For example, because light sources are placed at 10 degree intervals in this embodiment, the astigmatic axis angles can be presented in units of at least 10 degrees. In order to present the astigmatic axis angles, etc. with higher precision, the quantity of light sources, which corresponds to the precision (for example in units of 5 degrees, then 72 light sources), should be provided. The same can be said for lower precision cases.

Furthermore, though multiple light sources (the group of LEDs 131-i) are composed individually in this embodiment, this is not a restriction. For example, a display device (such as LCD (liquid-crystal display)) is provided on the bottom surface of the head part 131 and similar functionality can be obtained by displaying multiple bright points with this display device. In this case, each bright point corresponds to a "light source."

An objective lens part 16 is provided at the bottom end of the lens tube part 10. An objective lens 15 (as stated below) is stored in the objective lens part 16. The supporting member 17 is provided in the vicinity of the objective lens part 16. The supporting member 17 is formed laterally from the objective lens part 16.

At the tip 17a of the supporting member 17, a vertically extending open hole is formed. An arm 133 is inserted into this open hole. The arm 133 is slidable, inside the open hole. As a result, the arm 133 can be moved vertically (direction shown by the arrow A pointing at both sides in Fig. 2) with regard to the tip 17a. Here, the microscope 6 side is the top and the patient's eye E side is the bottom.

An anti-drop part 134 is provided at the top of the arm 133. The anti-drop part 134 is a plate-like member with a diameter bigger than the caliber of the open hole. As a result, the anti-drop part 134 prevents the arm 133 from falling from the tip 17a.

A head connecting part 132 is provided at the bottom end of the arm 133. The head connecting part 132 connects the head part 131 to the arm 133 such that the surface with LEDs 131-i face the bottom.

With such a configuration, the head part 131, the head connecting part 132, the arm 133, and the anti-drop part 134 (that is, the projection image forming part 13) can move freely and vertically with regard to the tip 17a. The projection image forming part 13 is moved by, for example, a user holding the arm 133, etc. Furthermore, by using a driving means such as a motor, the projection image forming part 13 can be composed such that it is moved electrically.

A connection hook 18 is provided on the bottom surface of the supporting member 17. The connection hook 18 is composed such that it can be engaged to an engaging part (not shown) of the projection image forming part 13. This engaging part is, for example, provided in the head connecting part 132. When the projection image forming part 13 is moved to the top, the engaging part engages the connection hook 18 to stop the vertical movement of the projection image forming part 13. This engagement relationship can be released by a specified operation (for example by pressing a specified button). The configurations of the connection hook 18 and the engaging part are optional.

With the above configuration, the group of LEDs 131-i is maintained such that they can move in the direction of the optical axis of the objective lens 15. Furthermore, the fixation 131 c is placed in the optical axis of the objective lens 15.

[Configuration of the optical system]

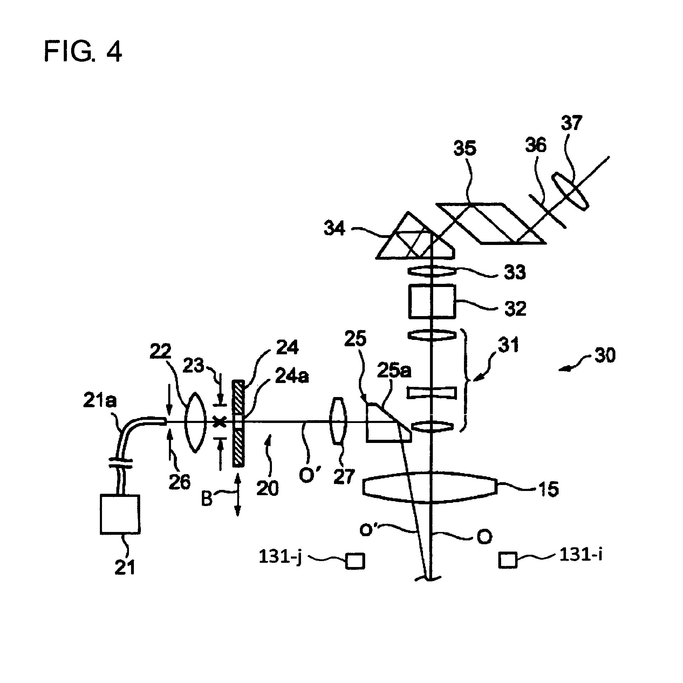

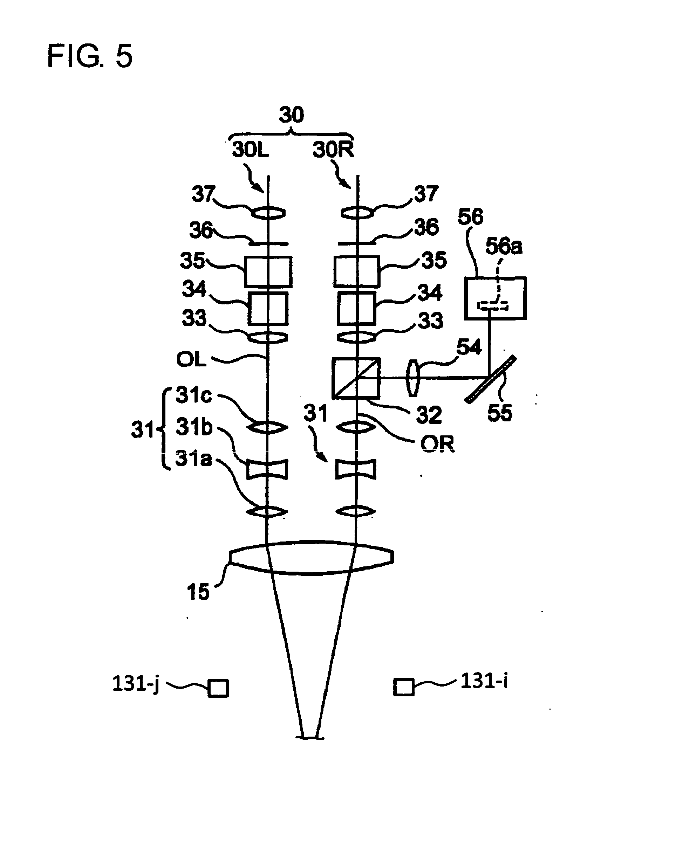

The optical system of the microscope for ophthalmologic surgery 1 will be explained next, with reference to Fig. 4 and Fig. 5. Here, Fig. 4 is a diagram, in which the optical system is viewed from the operator's left side. Moreover, Fig. 5 is a diagram, in which the optical system is viewed from the operator side. It should be noted that in addition to the configurations indicated in Fig. 4 and Fig. 5, an optical system (microscope for an assistant) for an assistant of the operator to observe the patient's eye E can be provided.

In this embodiment, directions such as top-and-bottom, left-and right, and front-and-back are directions viewed from the operator side unless otherwise specified. Moreover, with regards to the vertical direction, the direction from the objective lens 15 to the observation object (patient's eye E) is considered the lower direction while the opposite direction is considered the upper direction. Because patients generally go through an operation facing up, the top-and-bottom direction and the vertical direction become the same.

The group of LEDs 131-i is provided at the lower position of the objective lens 15 (that is, at the position between the objective lens 15 and the patient's eye E). Only LED 131-i and 131-j (i, j=1 to N, i≠j), which are positioned at both ends when viewed from the direction of this view point, are indicated in Fig. 4 and Fig. 5.

Moreover, a gap between the objective lens 15 and the patient's eye E means a gap between the position (height) of the objective lens 15 and the position (height) of the patient's eye E vertically (thus positions in the horizontal and front-and-back directions are not considered). Though the group of LEDs 131-i is placed in a ring, the diameter of this ring can be set arbitrarily as long as the size of an image of the light (bright point image) from the entire group of LEDs 131-i is projectable to the cornea of the patient's eye E.

An observation optical system 30 will be explained. A symmetrical pair of observation optical systems 30 is provided as indicated in Fig. 5. The observation optical system 30L on the left will be referred to as a left observation optical system and the observation optical system 30R will be referred to as a right observation optical system. A symbol OL indicates an optical axis (observation optical axis) of the left observation optical system 30L and a symbol OR indicates an optical axis (observation optical axis) of the right observation optical system 30R. The observation optical systems 30L and 30R on the left and right are arranged such that an optical axis O of the objective lens 15 is located in the middle of the optical axes OL and OR.

As before, each of the observation optical systems 30L and 30R on the left and right has a zoom lens system 31, a beam splitter 32 (only the right observation optical system 30R), an imaging lens 33, an image-erecting prism 34, a pupil-distance-adjusting prism 35, a field diaphragm 36, and an eyepiece lens 37.

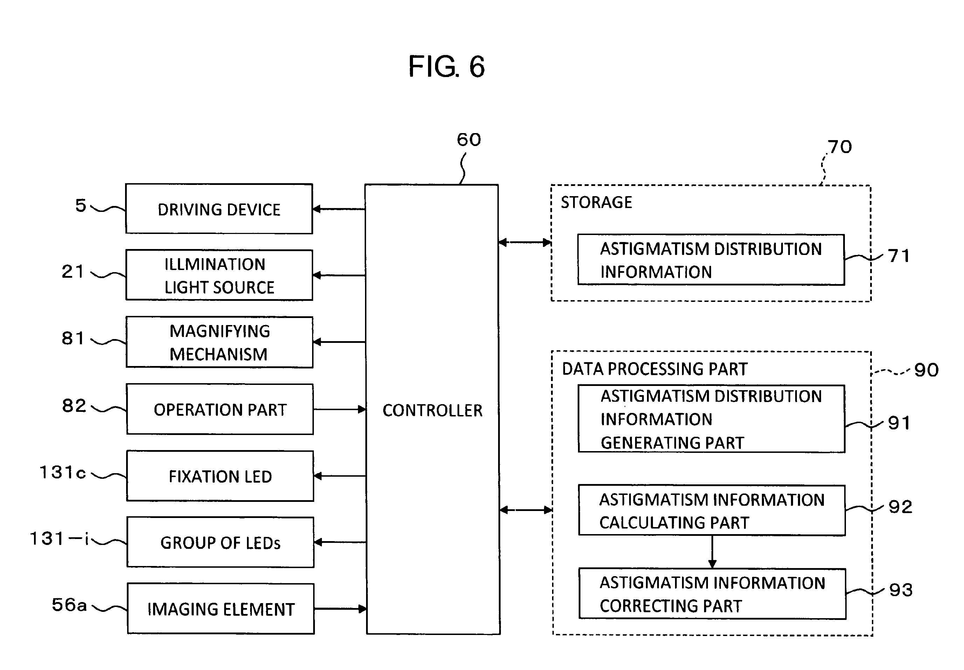

The zoom lens system 31 contains multiple zoom lenses 31a, 31b, and 31 c. Each zoom lens 31 a to 31 c is movable in the direction of the observation optical axis OL (or the observation optical axis OR) by the magnifying mechanism 81 that will be mentioned later (refer to Fig. 6). As a result, the magnification is changed when observing or photographing the patient's eye E.

The beam splitter 32 of the right observation optical system 30R separates the part of the observation light that has been guided along the observation optical axis OR from the patient's eye E and leads to the TV camera imaging system. The TV camera imaging system comprises an imaging lens 54, a reflecting mirror 55, and a TV camera 56. The TV camera imaging system is stored in the photographing part 14. An optical element that is placed in the optical path from objective lens 15 to the TV camera 56 (imaging element 56a) is an example of an "imaging optical system."

The TV camera 56 is equipped with an imaging element 56a. The imaging element 56a is, for example, composed of a CCD (Charge Coupled Devices) image sensor, CMOS (Complementary Metal Oxide Semiconductor) image sensor, etc. As the imaging element 56a, an element that has a two-dimensional light-receiving surface (that is, an area sensor) is used.

When using the microscope for ophthalmologic surgery 1, the light-receiving surface of the imaging element 56a is placed, for example, at an optically conjugated position with the corneal surface of the patient's eye E or at an optically conjugated position that is deep from the corneal apex by half of a corneal curvature radius.

The image-erecting prism 34 converts a reversed image to an erect image. The pupil-distance-adjusting prism 35 is an optical element for adjusting the distance between left and right observation lights in accordance with the pupil distance of the operator (the distance between left and right eyes). The field diaphragm 36 restricts the operator's field of vision by defilading the surrounding area in a cross-section of the observation light.

An illumination optical system 20 is explained next. As indicated in Fig. 4, the illumination optical system 20 comprises an illumination light source 21, an optical fiber 21a, an emission diaphragm 26, a condensing lens 22, an illumination field diaphragm 23, a slit plate 24, a collimator lens 27, and an illumination prism 25.

The illumination field diaphragm 23 is provided at a position that is optically conjugate to the front focal position of the objective lens 15. Furthermore, a slit hole 24a of the slit plate 24 is formed at the optically conjugate position against the front focal position.

The illumination light source 21 is provided outside the lens tube part 10 of the microscope 6. One end of the optical fiber 21a is connected to the illumination light source 21. The other end of the optical fiber 21a is placed at a position facing the condensing lens 22 inside the lens tube part 10. Illumination light that is output from the illumination light source 21 enters the condensing lens 22 after being guided by the optical fiber 21 a.

The emission diaphragm 26 is provided at a position facing the exit end (fiber edge on the condensing lens 22 side) of the optical fiber 21a. The emission diaphragm 26 works such that some area of the exit end of the optical fiber 21a is covered. When the covered area is changed by the emission diaphragm 26, the exit area of the illumination light is changed. As a result, for example, the projection angle of the illumination light, that is the angle formed by the incident direction of the illumination light toward the patient's eye E and the optical axis O of the objective lens 15, can be changed.

The slit plate 24 is formed by a discoidal member with a light blocking effect. A transparent part that is composed of multiple slit holes 24a with shapes corresponding to the shape of the reflective surface 25a of the illumination prism 25 is provided on the slit plate 24. The slit plate 24 is moved in a direction orthogonal to an illumination optical axis O' (in the direction shown by the arrow B pointing at both sides in Fig. 4) by a drive mechanism (not drawn). As a result, the slit plate 24 is inserted into and removed from the illumination optical axis O'.

The collimator lens 27 turns the illumination light that has passed through the slit hole 24a into parallel luminous flux. The illumination light that has been turned into the parallel luminous flux is reflected by the reflective surface 25a, of the illumination prism 25 and projected onto the patient's eye E via the objective lens 15. (Some of) the illumination light projected onto the patient's eye E is reflected by the cornea. The reflected light (sometimes referred to as observation light) of the illumination light by the patient's eye E enters the observation optical system 30 via the objective lens 15. Due to such a configuration, a magnified image of the patient's eye E becomes observable.

[Configuration of the control system]

The control system of the microscope for ophthalmologic surgery 1 will be explained with reference to Fig. 6. Moreover, the control system is partially shown in Fig. 6. The drive mechanism for the slit plate 24, etc. is omitted.

(Controller)

The control system of the microscope for ophthalmologic surgery 1 is composed around the controller 60. The controller 60 is provided at an arbitrary part of the microscope for ophthalmologic surgery 1. Furthermore, aside from the configuration indicated in Fig. 1, a computer and/or a circuit board can be provided and used as the controller 60. The controller 60 is composed of a microprocessor and a storage device similar to a normal computer.

The controller 60 controls each part of the microscope for ophthalmologic surgery 1. In particular, the controller 60 controls the driving device 5, the illumination light source 21, the magnifying mechanism 81, the group of LEDs 131-i, the fixation LED 131c, etc. The driving device 5 moves the microscope 6 3-dimensionally after being controlled by the controller 60. The magnifying mechanism 81 moves each of the zoom lenses 31a, 31b, and 31c of the zoom lens system 31 after being controlled by the controller 60. For example, a pulse motor is provided in the driving device 5 and the magnifying mechanism 81. The controller 60 controls the motion by sending pulse signals to each pulse motor.

(Storage)

All types of information are stored in the storage 70. In particular, the storage 70 stores astigmatism distribution information 71. The astigmatism distribution information 71 shows the distribution of the astigmatism parameters resulting from the power distribution of the imaging optical system at least. The power distribution of the imaging optical system indicates the power of the optical element composing the imaging optical system, which becomes the source of distortion of an image (such as distortion) that affects calculations of the astigmatism information of the patient's eye E. The distribution of the astigmatism parameters is a distribution in an image frame used in the calculation process of the astigmatism information of the patient's eye E. Furthermore, the astigmatism parameters, for example, include an astigmatic degree and an astigmatic axis angle.

In this embodiment, video of the patient's eye E while multiple bright points are projected is acquired and captured images of this video are used for the calculation process of astigmatism information. As a result, not only the power distribution of the imaging optical system but also the size difference between image frames photographed by the imaging element 56a and frames of the captured images become the source of image distortion. The aspect ratio of the imaging element 56a is an example of the frame size difference. For example, if a captured image with an aspect ratio of 16:9 is acquired from an image with an aspect ratio of 4:3, the image will be stretched horizontally.

Astigmatism distribution information 71 can be generated by actual measurements (sometimes referred to as preliminary measurements) and can also be generated theoretically or by a simulation based on required factors such as the configuration of the imaging optical system and aspect ratio. In this embodiment, a case that generates astigmatism distribution information 71 from preliminary measurements will be explained (mentioned later). Moreover, because the actual image is analyzed when astigmatism distribution information 71 is generated from preliminary measurements, it is difficult to specify how much and how the power of the imaging optical system and the aspect ratio are affected to produce images with such distortions. Nevertheless, by observing the patient's eye E and measuring astigmatism information with the same imaging optical system and the imaging element 56a as the preliminary measurements, it is natural to assume that the same distortion of the image during the preliminary measurements will result.

(Operation part)

The operation part 82 is used by an operator, etc. to operate the microscope for ophthalmologic surgery 1. The operation part 82 includes all types of hardware keys (buttons, switches, etc.) that are provided in a case, etc. of the microscope 6 as well as the foot switches 8. Furthermore, when a touch panel display and GUI are provided, various types of software keys displayed on these are included in the operation part 82.

(Data processing part)

The data processing part 90 executes various types of data processing. For example, the data processing part 90 calculates the astigmatic axis angle of the patient's eye E based on the image of the patient's eye E while luminous fluxes from the group of LEDs 131-i are projected onto the cornea. This process includes arithmetic processing similar to traditional keratometers, etc. Furthermore, the data processing part 4 generates astigmatism distribution information 71.
In order to execute such processes, the data processing part 90 has an astigmatism distribution information generating part 91, an astigmatism information calculating part 92, and an astigmatism information correcting part 93. The astigmatism distribution information generating part 91 is an example of a "generating part," the astigmatism information calculating part 92 is an example of a "calculating part," and the astigmatism information correcting part 93 is an example of a "correcting part."

(The astigmatism distribution information generating part)

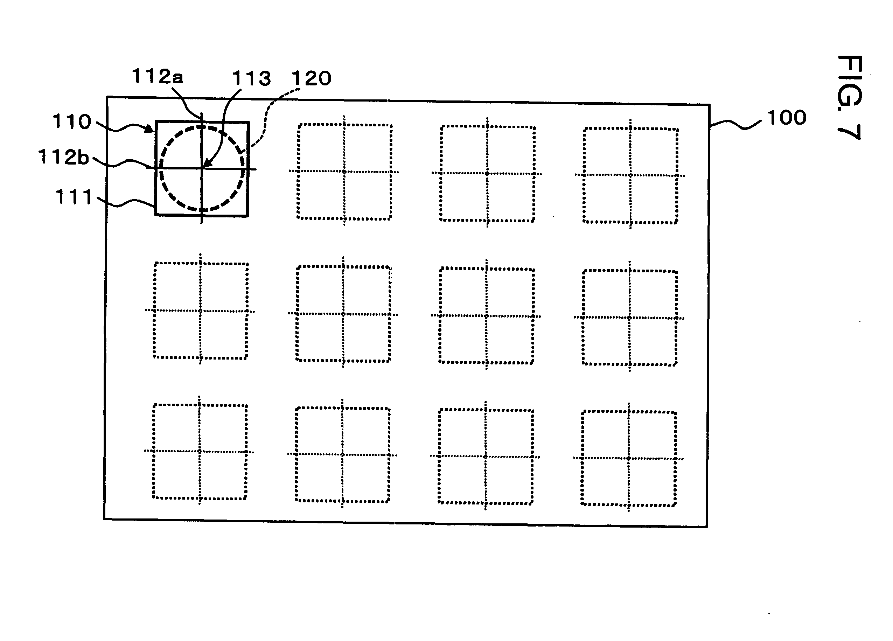

The astigmatism distribution information generating part 91 finds astigmatism parameters at each of multiple positions in an image frame based on an image photographed by the imaging element 56a and generates the astigmatism distribution information 71 by applying the least-squares method to the obtained multiple astigmatism parameters.
In this embodiment, the astigmatism distribution information generating part 91 finds multiple sets of the astigmatic degree and the astigmatic axis angle as the multiple astigmatism parameters based on multiple images on which an image of a schematic eye without astigmatism (astigmatism-free schematic eye) is depicted at each of multiple positions while multiple bright points are projected. A specific example of this process will be explained below.
Firstly, the above-mentioned preliminary measurement is performed. The astigmatism-free schematic eye is used in the preliminary measurement. When execution of the operation mode (calibration mode) for generating the astigmatism distribution information is directed, the controller 60 lights the illumination light source 21 as well as the group of LEDs 131-i and displays a specific screen on a display device (not shown). Moreover, the display device can be provided in the microscope for ophthalmologic surgery 1 itself and can also be directly or indirectly connected to the microscope for ophthalmologic surgery 1.
An example of this screen is indicated in Fig. 7. The area of a setup screen 100 corresponds to a frame on which images used for analysis processing for measuring astigmatism are displayed. A target 110 is overlaid on the setup screen 100. The target 110 is composed of a rectangular frame 111 as well as a vertical line 112a and a horizontal line 112b which cross at the center 113 of the rectangular frame 111.
The user displays the target 100 in a desired position on the setup screen 110 by using the operation part 82 or an external operation device. The controller 60 or an external microprocessor displays, on the setup screen 100, a video of the astigmatism-free schematic eye while light from the group of LEDs 131-i is projected.
The user adjusts the relative position of the astigmatism-free schematic eye as well as the optical system of the microscope for ophthalmologic surgery 1 and displays the bright point images from the group of LEDs 131-i on the target 100. At this time, the center of the multiple bright point images 120 arranged in a circle or ellipse should be matched with the center 113 of the rectangular frame 111. When the user directs photographing using the operation part 82, the astigmatism distribution information generating part 91 generates a captured image of a video of the astigmatism-free schematic eye.
The user performs such processing at multiple positions on the setup screen 100. All the positions on the setup screen 100 are specified by, for example, the position of the center 113 of the rectangular frame 111. Moreover, multiple display positions of the target 100 can be decided beforehand and the targets 100 can be displayed in order at these display positions corresponding to a specific trigger.
The number of times the processing such as above is executed is decided depending on the degree of the least square approximation mentioned later. For example, the process is executed at 9 positions or more when quadratic approximation is performed and at 17 positions or more when cubic approximation is performed. Moreover, positions are not limited to the 12 positions shown in Fig. 7; however this is just an example.

Based on the processing above, multiple captured images on which bright point images 120 are drawn at multiple positions on the setup screen 100 can be obtained. The astigmatism distribution information generating part 91 generates the astigmatism distribution information 71 by executing the following processing based on these captured images. Moreover, the astigmatism distribution information 91 can be provided to an external computer of the microscope for ophthalmologic surgery 1.

First, the astigmatism distribution information generating part 91 finds astigmatism parameters by performing known arithmetic processing similar to keratometers, etc. based on each captured image. The astigmatism parameters are, for example, a combination of the astigmatic degree and the astigmatic axis angle at the center position of an arrangement of the bright point images (generally elliptic array) in the captured image resulting from the power distribution of the imaging optical system and aspect ratio. The astigmatic degree is shown as $C_i$ and the astigmatic axis angle is shown as $\theta_i$ corresponding to each of the multiple (N) captured images (i=1 to N). Processing that calculates the astigmatism parameters based on each captured image can be performed by the astigmatism information calculating part 92. Moreover, although the following example explains a case of quadratic approximation, cases of cubic or more degree approximation is similar.

The astigmatism distribution information generating part 91 finds a two-dimensional orthogonal coordinates $(X_i, Y_i)$ corresponding to polar coordinates $(C_i, \theta_i)$ with the astigmatic degree $C_i$ as the radius vector as well as the doubled value $2\theta_i$ of the astigmatic axis angle $\theta_i$ as a polar angle with regard to astigmatism parameters corresponding to each captured image. Here, the polar coordinates $(C_i, \theta_i)$ are coordinates of the polar coordinate system $(C_i, \theta_i)$ and the two-dimensional orthogonal coordinates $(X_i, Y_i)$ is made up of coordinates of a two-dimensional orthogonal coordinate system $(X, Y)$. The following formula can be obtained as a result.

$$[\mathrm{Formula~1}]$$

$$X_i = C_i \times \cos 2\theta_i$$

$$Y_i = C_i \times \sin 2\theta_i$$

As a result, the X component and Y component corresponding to each captured image can be obtained. Moreover, the astigmatism distribution information generating part 91 calculates the coordinates $(x_i, y_i)$ of the center of the elliptic array of the bright points for each captured image. The coordinates $(x_i, y_i)$ of the center can be obtained, for example, by calculating the crossing position of the major axis and the minor axis of an ellipse or as coordinates of the center 113 of the rectangular frame 111.

Subsequently, the astigmatism distribution information generating part 91 calculates coefficients a, b, c, d, e and h in the following formula that is based on X component $X_i$ and coordinates $(x_i, y_i)$ of the center corresponding to each captured image using the least square approximation.

$$[\mathrm{Formula~2}]$$

$$X_i(x, y) = ax^2 + by^2 + hxy + cx + dy + e$$

Similarly, with regard to the Y component $Y_i$ and coordinates $(x_i, y_i)$ of the center, the astigmatism distribution information generating part 91 calculates coefficients in the following formula using a least square approximation.

$$[\mathrm{Formula~3}]$$

$$Y_i(x, y) = a'x^2 + b'y^2 + h'xy + c'x + d'y + e'$$

These coefficients are calculated in the following manner. As a specific example, the astigmatism distribution information generating part 91 generates astigmatism distribution information 71 by executing the following process:

(1) Find a two-dimensional orthogonal coordinates $(X_i, Y_i)$ corresponding to polar coordinates $(C_i, \theta_i)$ that has the astigmatic degree $C_i$ as the radius vector as well as the doubled value $2\theta_i$ of the astigmatic axis angle $\theta_i$ as a polar angle with regard to each multiple astigmatism parameter (a combination of the astigmatic degree and the astigmatic axis angle);
(2) Apply the least-squares method to multiple coordinates $X_i$ in the multiple two-dimensional orthogonal coordinates $(X_i, Y_i)$ and calculate the first nth approximation of the surface of the entire frame (setup screen 100);
(3) Apply the least-squares method to multiple coordinates $Y_i$ in the multiple two-dimensional orthogonal coordinates $(X_i, Y_i)$ and calculate the second nth approximation of the surface of the entire frame (setup screen 100);
(4) The first nth approximation of the surface and the second nth approximation of the surface are defined as the astigmatism distribution information 71.

The astigmatism distribution information 71 obtained in this way is stored in the storage 70 by the controller 60. The astigmatism parameters at each position in the frame are recorded in the astigmatism distribution information 71. More specifically, the astigmatism distribution information 71 is information obtained by applying the least-squares method to measurement results of astigmatism parameters at multiple positions in the frame (N positions). The measurement results of the astigmatism parameters are coordinates $X_i$ and $Y_i$, which are calculated from the formula (1) with the astigmatic degree $C_i$ and the astigmatic axis angle $\theta_i$ at each position. The astigmatism distribution information 71 can be obtained by applying the least-squares method to the coordinates $X_i$ and $Y_i$, in order to expand its domain from the discrete n points to the entire frame. That is, the astigmatism distribution information 71 is expressed as a vector field in which the domain is defined for the t entire frame and a vector is given by $(X_c, Y_c)$. Moreover, the astigmatism distribution information 71 is not limited to this and its form is optional as long as it at least shows the distribution of the astigmatism parameters resulting from the power distribution of the imaging optical system. For example, the above coefficients a to h can be used as astigmatism parameters, and n polar coordinates $(C_i, \theta_i)$ or the n two-dimensional orthogonal coordinates $(X_i, Y_i)$ can be used as astigmatism parameters. In these cases, the astigmatism information correcting part 93 mentioned later corrects measured values of the astigmatic degree and the astigmatic axis angle of the patient's eye E by properly using the formula above.

Instead of finding the nth approximation surface of the entire frame (setup screen 100) in the processes (2) and (3), an nth approximation surface defined on only a part of the frame can be obtained. For example, because an image of the patient's eye E (that is bright point images) will not be (at least rarely) displayed in the vicinity of an edge of the frame, it is not a (at least small) problem to find only the nth approximation surface of the area excluding the area in the vicinity of the edge of the frame. The area in the vicinity of the edge of the frame can be preliminary determined or can also be determined depending on factors such as observation magnification.

(Astigmatism information calculating part)

The astigmatism information calculating part 92 calculates the astigmatism information based on the images obtained by photographing the patient's eye E with imaging element 56a while multiple bright points are projected. More specifically, the astigmatism information calculating part 92 finds the astigmatic degree and the astigmatic axis angle based on an array of the multiple bright point images which are depicted in a photographed image (a captured image) of the patient's eye E by executing a known arithmetic processing similar to keratometers, etc. That is, the astigmatism information calculating part 92 calculates the astigmatic degree from the ellipticity (ratio of the minor axis and the major axis) of elliptically arranged multiple bright point images, and calculates information of the astigmatic axis angle from the direction of the main axis of the ellipse. Furthermore, sphericity can be found from the size of the ellipse.

(Astigmatism information correcting part)

The astigmatism information correcting part 93 corrects astigmatism information calculated by the astigmatism information calculating part 92 based on the astigmatism distribution information 71. The following correction process is executed in this embodiment:

(1) Find the two-dimensional orthogonal coordinates $(X_m, Y_m)$ corresponding to polar coordinates $(C, \theta)$ with an astigmatic degree C that has been calculated by the astigmatism information calculating part 92 as the radius vector as well as the doubled value $2\theta$ of the astigmatic axis angle $\theta$ as a polar angle (similar process as the astigmatism distribution information generating part 91);
(2) Specify the coordinate $X_c$ of the first approximation surface of nth order and coordinates $Y_c$ of the second approximation surface of nth order at the center position of an elliptic array of the multiple bright point images depicted in the image of the patient's eye E obtained by the imaging element 56a, and subtract the specified coordinate $X_c$ and coordinate $Y_c$ from the coordinate $X_m$ and coordinate $Y_m$ , respectively;

(3) Transform the coordinate $X_m$ -$X_c$ and the coordinate $Y_m$ -$Y_c$ obtained by this subtraction process to the polar coordinates.

This process is explained in detail. When measurement of the astigmatism of the patient's eye E is carried out, the astigmatism information correcting part 93 finds the center position (x, y) of the elliptic array of the multiple bright point images depicted in the image of the patient's eye E, and obtains the astigmatism parameters ($X_c$, $Y_c$) at this center position (x, y) from the astigmatism distribution information 71. Furthermore, the astigmatism information correcting part 93 calculates the two-dimensional orthogonal coordinates ($X_m$, $Y_m$) corresponding to the astigmatic degree C and the astigmatic axis angle $\theta$ (polar coordinates (C, $\theta$)) calculated by the astigmatism information calculating part 92 similar to the [formula 1]. Consequently, the astigmatic degree and the astigmatic axis angle of the patient's eye E are normalized using the following formula.

$$[\text{Formula 4}]$$

$$X = X_m - X_c$$
$$Y = Y_m - Y_c$$

The astigmatism information correcting part 93 calculates the correction value (C', $\theta$') of the measured value (C, $\theta$) of the astigmatism information of the patient's eye E by substituting the normalized X component and Y component into the following formula.

$$[\text{Formula 5}]$$

$$C' = \sqrt{X^2 + Y^2}$$

$$\theta' = \frac{1}{2}\tan^{-1}\left(\frac{Y}{X}\right)$$

The controller 60 specifies LED 131-i at the position corresponding to the astigmatic axis angle $\theta$' in the correction value (C', $\theta$') obtained this way from the group of LEDs 131-i. Furthermore, the controller 60 lights specified LED 131-i in a different aspect from other LED 131-j (j$\neq$i). An example of this process is written in Japanese published unexamined application 2011-110172.

[Actions and effects]

The actions and effects of the microscope for ophthalmologic surgery 1 above will be explained.

The astigmatism distribution information 71 that at least expresses a distribution of astigmatism parameters resulting from a power distribution of the imaging optical system is stored in the storage 70 of the microscope for ophthalmologic surgery 1 in advance. "In advance" means before measuring the astigmatism of the patient's eye E. When measurement of the patient's eye E is performed, the astigmatism information calculating part 92 calculates astigmatism information based on the images obtained by photographing with the imaging element 56a the patient's eye E while light from the group of LEDs 131-i is being projected. Furthermore, the astigmatism information correcting part 93 corrects this astigmatism information based on the astigmatism distribution information 71.

According to such a microscope for ophthalmologic surgery 1, distortions of images resulting from the power distribution of the imaging optical system, etc. can be corrected. Furthermore, in this embodiment, not only the power distribution of the imaging optical system but also distortions of images resulting from the aspect ratio can be corrected. Furthermore, the size of image distortions by power distributions of the imaging optical system and aspect ratios change depending on the position in the frame. As a result, according to this embodiment, astigmatic axis angles can be measured with high accuracy regardless of the depicted position of the patient's eye E in the image frame.

Furthermore, according to this embodiment, an aspect of LED 131-i at a position corresponding to a correction value of the astigmatic axis angle can be different from the aspect of other LED 131-j. As a result, an operator can visually and intuitively recognize the astigmatic axis angle (value that has been corrected with high accuracy) of the patient's eye E while observing the patient's eye E. Consequently, transplant surgery of the toric IOL, etc. can be simplified.

Furthermore, astigmatism parameters at each position in the frame are recorded in the astigmatism distribution information 71. As a result, the above correction can be performed based on multiple bright point images depicted at any positions in the frame.

Furthermore, information obtained by applying the least-squares method to measurement results of the astigmatism parameters at multiple positions in the frame is recorded as the astigmatism distribution information 71. As a result, astigmatism parameters regarding a two-dimensional area in the frame can be obtained from the astigmatism parameters at multiple positions in the frame. Furthermore, because the least-squares method is used, the approximation error is also small. Moreover, in order to decrease approximation errors, the degree of approximation formula should be increased. When the degree is increased, however, the number of measurement positions at preliminary measurements increases and the amount of data spent on processing and processing time increases. Consequently, it is desirable that the degree be decided after taking these various factors into account.

[Modification example]

The above embodiment is just an example of the microscope for ophthalmologic surgery related to the present invention. Those intending to work the present invention can carry out any modifications within the scope of the present invention.

Though multiple bright points placed in a ring are used in the preliminary measurements of the above embodiment, there are no limitations on the use. For example, by projecting a grid-like bright line group made up of by crossing multiple vertical lines and multiple horizontal lines, distribution of image distortions appearing in the frame (that is, distribution of astigmatism parameter) can be found. Generally, if distribution of astigmatism parameters can be found, methods of the preliminary measurements are optional.

Furthermore, the processing that generates astigmatism distribution information 71 and the processing that corrects astigmatism information are not limited to the processes described above. In so far as achieving each purpose, the specific processing details are optional.

[Explanation of Symbols]

1 Microscope for ophthalmologic surgery
5 Driving device
13 Projection image forming part
20 Illumination optical system
21 Illumination light source
30 Observation optical system
56a Imaging element
70 Storage
71 Astigmatism distribution information
81 Magnifying mechanism
82 Operation part
90 Data processing part
131-i LED
131 c fixation LED

**Claims**

1. A microscope for ophthalmologic surgery comprising:

    an optical system that includes an illumination optical system that illuminates the patient's eye and an imaging optical system that has an imaging element that photographs said illuminated patient's eye;
    a main body part that stores at least a part of said optical system;

an attachment that is attached to said main body part and has multiple bright points arranged in a ring;

a calculating part that calculates astigmatism information based on images obtained by photographing said patient's eye with said imaging element while said multiple bright points are being projected;

a storage in which astigmatism distribution information that shows the distribution of astigmatism parameters resulting from the power distribution of said imaging optical system at least in advance; and

a correcting part that corrects the astigmatism information calculated by said calculating part based on said astigmatism distribution information.

**2.** The microscope for ophthalmologic surgery according to Claim 1,
wherein
said astigmatism information calculated by said calculating part includes the astigmatic axis angle,
and further comprises a controller that specifies a bright point at a position corresponding to said corrected astigmatic axis angle from among said multiple bright points and makes the aspect of said specified bright point different from the aspect of other bright points.

**3.** The microscope for ophthalmologic surgery according to Claim 1 or Claim 2, wherein said astigmatism parameters at each position in a frame of a photographed image by the imaging element is recorded in said astigmatism distribution information.

**4.** The microscope for ophthalmologic surgery according to any one of Claims 1 through Claim 3, wherein information obtained by applying a least-squares method to the measurement results of said astigmatism parameters at multiple positions in a frame of a photographed image by said imaging element is recorded in said astigmatism distribution information.

**5.** The microscope for ophthalmologic surgery according to any one of Claims 1 through Claim 4, further comprising a generating part that obtains, based on a photographed image by said imaging element, said astigmatism parameters at each of multiple positions in the relevant frame, and generates said astigmatism distribution information by applying the least-squares method to the obtained multiple astigmatism parameters.

**6.** The microscope for ophthalmologic surgery according to Claim 5, wherein said generating part obtains multiple pairs of an astigmatic degree and an astigmatic axis angle as said multiple astigmatism parameters based on multiple said photographed images on which an image of an astigmatism-free schematic eye is drawn at each of said multiple positions while said multiple bright points are being projected.

**7.** The microscope for ophthalmologic surgery according to Claim 6, wherein said generating part:

obtains, with regard to each of said pair of the astigmatic degree and the astigmatic axis angle, two-dimensional orthogonal coordinates $(X_i, Y_i)$ corresponding to polar coordinates $(C_i, \theta_i)$ that treat said astigmatic degree $C_i$ as a radius vector and the doubled value $2\theta_i$ of said astigmatic axis angle $\theta_i$ as a polar angle;

calculates a first approximation surface of nth order for partial or the entire said frame by applying the least-squares method to the multiple coordinates $X_i$ in the obtained multiple two-dimensional orthogonal coordinates $(X_i, Y_i)$; and

calculates the second approximation surface of nth order for partial or the entire said frame by applying the least-squares method to the multiple coordinates $Y_i$ in the multiple two-dimensional orthogonal coordinates $(X_i, Y_i)$,

wherein said calculated first approximation surface of nth order and said second approximation surface of nth order are treated as said astigmatism distribution information.

**8.** The microscope for ophthalmologic surgery according to Claim 7,
wherein
said calculating part calculates the astigmatic degree and the astigmatic axis angle as said astigmatism information, and
said correcting part:

obtains the two-dimensional orthogonal coordinates $(X_m, Y_m)$ corresponding to polar coordinates $(C, \theta)$ that treat the astigmatic degree C calculated by said calculating part as the radius vector and the doubled value $2\theta$ of the astigmatic axis angle $\theta$ as a polar angle;

subtracts coordinates $X_c$ of said first approximation surface of nth order and coordinates $Y_c$ of said second approximation surface of nth order at the center position of an elliptic array of the images of said multiple bright points which are drawn in an image of said patient's eye obtained by said imaging element, from said coordinates $X_m$ and said coordinates $Y_m$, respectively; and

obtains the astigmatic degree and the astigmatic axis angle by transforming the coordinates $X_m - X_c$ and the coordinates $Y_m - Y_c$ obtained from the subtraction into polar coordinates.

FIG. 1

# FIG. 2

FIG. 3

EP 2 550 912 A1

FIG. 4

18

## FIG. 5

# FIG. 6

DRIVING DEVICE — 5

ILLMINATION LIGHT SOURCE — 21

MAGNIFYING MECHANISM — 81

OPERATION PART — 82

FIXATION LED — 131c

GROUP OF LEDs — 131−i

IMAGING ELEMENT — 56a

CONTROLLER — 60

STORAGE — 70

ASTIGMATISM DISTRIBUTION INFORMATION — 71

DATA PROCESSING PART — 90

ASTIGMATISM DISTRIBUTION INFORMATION GENERATING PART — 91

ASTIGMATISM INFORMATION CALCULATING PART — 92

ASTIGMATISM INFORMATION CORRECTING PART — 93

EP 2 550 912 A1

# FIG. 7

Europäisches
Patentamt

European
Patent Office

Office européen
des brevets

# EUROPEAN SEARCH REPORT

Application Number

EP 12 00 5109

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | JP 2011 110172 A (TOPCON CORP) 9 June 2011 (2011-06-09) * figures 1-6 * * paragraphs [0001], [0022] - [0024], [0047] - [0048], [0074] - [0075] * ----- | 1-8 | INV. A61B3/103 ADD. A61B3/13 G02B21/00 A61F2/16 |
| Y | EP 1 321 095 A2 (TOPCON CORP [JP]) 25 June 2003 (2003-06-25) * figure 20 * * paragraphs [0057] - [0066] * ----- | 1-8 | |
| Y | JP 2007 215956 A (TOMEY CORP) 30 August 2007 (2007-08-30) * paragraph [0020] * ----- | 1-8 | |
| A | US 2009/059169 A1 (SHIMIZU KAZUNARI [JP] ET AL) 5 March 2009 (2009-03-05) * paragraph [0032] * ----- | 4-8 | |

TECHNICAL FIELDS
SEARCHED (IPC)

A61B
G02B
A61F

The present search report has been drawn up for all claims

| Place of search | Date of completion of the search | Examiner |
|---|---|---|
| The Hague | 1 November 2012 | Albrecht, Ronald |

EPO FORM 1503 03.82 (P04C01)

**ANNEX TO THE EUROPEAN SEARCH REPORT
ON EUROPEAN PATENT APPLICATION NO.**

EP 12 00 5109

This annex lists the patent family members relating to the patent documents cited in the above-mentioned European search report.
The members are as contained in the European Patent Office EDP file on
The European Patent Office is in no way liable for these particulars which are merely given for the purpose of information.

01-11-2012

| Patent document cited in search report | | Publication date | Patent family member(s) | | Publication date |
|---|---|---|---|---|---|
| JP 2011110172 | A | 09-06-2011 | JP 2011110172 A | | 09-06-2011 |
| | | | WO 2011064943 A1 | | 03-06-2011 |
| EP 1321095 | A2 | 25-06-2003 | AT 364346 T | | 15-07-2007 |
| | | | CN 1423150 A | | 11-06-2003 |
| | | | DE 60220630 T2 | | 14-02-2008 |
| | | | EP 1321095 A2 | | 25-06-2003 |
| | | | JP 3856691 B2 | | 13-12-2006 |
| | | | JP 2003172876 A | | 20-06-2003 |
| | | | US 2003128333 A1 | | 10-07-2003 |
| JP 2007215956 | A | 30-08-2007 | JP 4859479 B2 | | 25-01-2012 |
| | | | JP 2007215956 A | | 30-08-2007 |
| US 2009059169 | A1 | 05-03-2009 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011110172 A